# EUROPEAN PATENT APPLICATION

(11) **EP 1 835 031 A1**
(43) Date of publication of application: **19.09.2007**
(21) Application number: 06005164.6
(22) Date of filing: 14.03.2006
(51) Int. Cl.: C12N 15/863, A61P 37/08, A61K 35/76, A61K 39/35, A61K 48/00, C12N 7/01

(54) **Use of a recombinant modified vaccinia virus Ankara (MVA) for the treatment of type I hypersensitivity in a living animal including humans**

(71) Applicant: Paul-Ehrlich-Institut Bundesamt für Sera und Impfstoffe, 63225 Langen (DE)
(72) Inventor: Albrecht, Melanie, 64390 Erzhausen (DE); Sutter, Gerd, 80803 München (DE); Süzer, Yasemin, 63110 Rodgau (DE); Reese, Gerald, 63225 Langen (DE); Vieths, Stefan, 63303 Dreieich (DE); Staib, Caroline, 80803 München (DE)
(74) Representative: Vossius & Partner

(57) **Abstract**

The present invention relates to the use of a recombinant modified vaccinia virus Ankara (MVA) comprising a heterologous nucleic acid for the production of a medicament for the prevention and/or treatment of type I hypersensitivity in a living animal including humans. The invention further relates to a recombinant modified vaccinia virus Ankara (MVA) comprising a heterologous nucleic acid, wherein the heterologous nucleic acid is incorporated into a non-essential region of the genome of the MVA, the heterologous nucleic acid is under the control of, e.g. a vaccinia virus-specific promoter and, the heterologous nucleic acid is selected from the group of nucleic acids encoding an allergen selected from the group of weed pollens, grass pollens, tree pollens, mites, animals, fungi, insects, rubber, worms, human autoallergens, and foods.

## Description

### Technical field

The present invention relates generally to the fields of chemistry, biology, biochemistry, molecular biology, in particular animal and human therapeutics, more in particular human vaccination.

The invention provides for novel recombinant modified vaccinia virus Ankara for the prevention and/prevention treatment of type I hypersensitivity in a living animal and humans.

The invention extends into the field of mammalian therapeutics and drug development, more in particularly into the field of vaccine therapeutics and vaccine drugs development.

### Introduction

Almost 500 million individuals suffer from type I allergy, a genetically determined hypersensitivity disease, which is based on the formation of IgE antibodies against *per se* harmless antigens (allergens) (Kay, A. B. (1997), Allergy and Allergic Diseases, Blackwell Signs, Oxford, UK). The symptoms of type allergy (allergic rhinitis, conjunctivitis and allergic asthma) are mainly caused by the allergen-mediated cross-linking of cell bound specific IgE antibodies and the consecutive release of biological mediators (e.g. histamine, leukotriens) (Segal, D. M., Taurog, J. P., and Metzger, H. (1977). Dimeric Immunoglobulin E Serves as a Unit Signal for Mast Cell Degranulation. Proc. Natl. Acad. Sci. USA 42, 457-467). Allergens may be weeds, such as Cariophylales, Gentianales, Lamiales, Rosales, grasses, such as Poales, trees, such as Arezales, Fagales, Lamiales, Pinales, Plactanmarceae, mites such as American house dust mite, house dust mite, European house dust mite, storage mite, animals such as domestic cattle, dog, domestic horse, cat, guinea pig, mouse, rat, fungi such as Ascomyzota, Eurotiales, Hypocreales, Onygenales, insects, such as honeybee, German cockroach, American cockroach, foods such as cod, salmon, cattle, chicken, shrimp and other Crustaccea, squid, snail, mussle, oyster and other Mollusca, nuts such as cashew and fruits such as the papaya. In fact, an allergy may also be caused by rubbers, such as latex.

Grasses and corn are distributed worldwide, produce large amounts of pollen which become easily airborne and therefore belong to the most important allergen sources. More than 40 % of allergic patients are sensitised against grass pollen allergens of which group two allergens represent one of the most frequently recognized allergens (Anvari, A. A., Shen Bagamurthi, P., and Marsch, D. G. (1989). Complete Primary Structure of a Lolium Perenneae (Perennial Grass) Pollen allergen, Lol p 3I: Comparison with known Lol p 1I and Lol p 2I sequences. Biochem. 28, 8665-8670).

Specific therapy of type I allergy can be achieved in principle by active and passive vaccination. Active vaccination is achieved by specific immunotherapy in order to induce unresponsiveness towards allergens. Although successfully practiced since 1911, the immunological mechanisms of specific immunotherapy are not completely understood (Noon, L. (1911). Prophylactic Innoculation against Hay Fever. Landset 1, 1572-1573; Bousquet, J., Lockay, R., Mulling, H. J., and the WHO Panel Members (1998). Allergen Immune Therapy: Therapeutic Vaccines for Allergic Diseases. A WHO position paper. J. Allergy Clin. Immunol. 102, 558-562). Induction of blocking antibodies of the IgG class which interfere with the IgE allergen interaction, modulation of T cell and effector cell responses and generation of tolerance are discussed as possible mechanisms (Durham, and S. R., Till, S. J. (1998). Immunological Changes associated with Allergen Immunotherapy. J. Allergy Clin. Immunol. 102, 157-164). In contrast to active vaccination, passive vaccination is based on the transfer of protective immunoglobulins and represents a routine treatment for many infectious diseases (e.g. hepatitis). The therapeutical efficacy of "passive vaccination" for the treatment of type I allergy has been demonstrated by classical experiments more than 60 years ago. 1935 Cooke and colleagues reported cure of a hay fever patient by transfer of blood from a patient who had been successfully treated by specific immunotherapy (Cooke, R. A., Bernhard, J. H., Hebald, S., and Stull, A. (1935). Serological Evidence of Immunity with CoExisting Sensitization in Hay Fever Type of Human Allergy. J. Exp. Med. 62, 733-750).

Today, a method called specific immune therapy (SIT) is often applied in order to cope with type I allergies. Here, an extract comprising e.g. pollen allergens or individual allergen is given subcutaneously or sublingually to a patient. The problem with SIT is, that the composition of natural allergen extracts is bound to be subject to natural fluctuations. Thus, optimal dosing of the allergen molecule within the extract is a difficult task. Also, this type of immunotherapy often goes along with substantial side-effects. Therefore, this type of immunotherapy may not be used for particular allergies such as food allergies. Additionally, SIT requires a high number of immunizations.

Thus, it would be advantageous to have a medicament for the treatment of type I hypersensitivity with little or no side-effects and which would require only very few administrations.

SIT may not be applied to particular types of allergies such as mould allergies or food allergies. Thus, it would be advantageous to have a medicament for the treatment of type I hypersensitivities which in particular could be applied to mould or food allergies.

The production of natural allergen extracts is difficult. Thus, it would be advantageous to have a medicament for the treatment of type I hypersensitivities which would be easy to produce.

Traditional SIT usually leads initially to a T_{H2}-response and consequently after further treatment to a switch from a T_{H2}-response to a T_{H1}-response. Thus, it would be advantageous to have available a medicament for the treatment of type I hypersensitivities resulting primarily in a T_{H1}-response.

WO 2003/088994 discloses a modified vaccinia virus Ankara for the vaccination of neonates. EP 1 518 932 A1 is directed to a MVA mutant and its use in the immunotherapy and vaccination against numerous diseases, in particular in the prevention and therapy of cancer and infectious diseases.

WO 03/097844 A1 concerns recombinant modified vaccinia virus Ankara comprising a viral genome and expression cassette comprising the cow pox ATI promoter or a derivative thereof and a coding sequence, wherein the expression of the coding sequence is regulated by said promoter. The virus disclosed in WO 03/097844 A1 is supposedly useful as a vaccine or as part of a pharmaceutical composition.

US 6,171,591 B1 discloses nodavirus related compositions. The chimeric virus particles are useful in therapeutic applications, such as vaccines and gene-delivery vectors, and in diagnostic applications, such as kits for the testing of body tissue or fluid samples.

WO 01/68820 A1 relates to new strains of the modified vaccinius virus Ankara (MVA) that have strongly reduced virulence for most mammals, especially humans, but nevertheless grow in cells of a continuous cell line approved for the production of a therapeutic agent such as a vaccine.

The inventors have been able to solve the above-identified problems by providing for a recombinant vaccinia virus Ankara (MVA).

### Brief description of the invention

The invention relates to the use of a recombinant modified vaccinia virus Ankara (MVA) comprising a heterologous nucleic acid for the production of a medicament for the prevention and/or treatment of type I hypersensitivity in a living animal including humans.

The inventors have astonishingly found that a recombinant modified vaccinia virus Ankara (MVA) comprising a heterologous nucleic acid may be advantageously used for the treatment of type I hypersensitivity.

Such a recombinant modified vaccinia virus Ankara (MVA) according to the invention comprises a heterologous nucleic acid, wherein the heterologous nucleic acid is incorporated into a non-essential region of the genome of the MVA, the heterologous nucleic acid is under the control of a vaccinia virus-specific promoter, orthopox virus-specific promoter, poxvirus-specific promoter, and the heterologous nucleic acid is selected from the group of nucleic acids encoding an allergen selected from the group of weed pollens, grass pollens, tree pollens, mites, animals, fungi, insects, rubber, worms, human autoallergens, and foods.

The invention further relates to a method of introducing MVA according to the invention into a target cell comprising infection of the target cell with the inventive MVA.

The invention further relates to a method for immunization of a living animal body including a human, said method comprising administering to said living animal body including a human in need thereof a therapeutically effective amount of the MVA according to the invention.

### Detailed description of the invention

The invention relates to the use of a recombinant modified vaccinia virus Ankara (MVA) comprising a heterologous nucleic acid for the production of a medicament for the prevention and/or treatment of type I hypersensitivity in a living animal including humans.

As outlined above, there are certain type I hypersensitivities, i.e. allergies which are very difficult to treat with the specific immunotherapy treatment (SIT). The inherent problem with SIT is that the mixture of natural allergen extract is bound to be subject to natural fluctuations. Also, SIT is known for substantial allergic side-effects. The recombinant modified vaccinia virus Ankara according to the present invention leads to an endogenous expression of the antigen, *i.e.* the heterologous nucleic acid, encoding the antigen in the host cell, which leads to a direct and preferred MHC I-restricted antigen presentation associated with a TH1-associated T helper cell response. Further, the inventors have found that for the strong immunogenicity of the inventive MVA as well as the strong immunomodulatory activity of the inventive MVA only few immunizations are necessary for a beneficial modulation of the immune response. Further, the inventors have astonishingly found that the MVA according to the present invention allows for preventive allergy vaccination which is so far not possible.

The MVA according to the present invention infects cells which produce the antigen. This leads to a predominantly MHC I restricted antigen presentation on the cell surface associated with a strong T_{H1} immune response against the allergen. Most advantageously, the MVA according to the invention may not replicate in animals or humans, which in turn leads to a lack of propagation and a high safety profile. Most advantageously, this effect makes it possible in a clinical environment to use the most efficient doses of the MVA according to the invention. Also, the MVA according to the invention may be used in a clinical and laboratory environment with the lowest safety rating (L1, S1).

In one embodiment the medicament according to the invention is a vaccine for immunotherapy and the immune response following application of said vaccine to said humans is primarily a T_{H1} response in humans. As outlined above, the inventors have astonishingly found, that the MVA leads to an advantageous T_{H1} response.

Proliferating helper T cells that develop into effector T cell differentiate into two major subtypes of cells known as T_{H1} and T_{H2} cells (also known as Type I and Type II helper T cells respectively). These subtypes are defined on a basis of the specific cytokines they produce. T_{H1} produce interferon-γ (or IFN-γ) and lymphotoxin (also known as tumor necrosis factor-β or TNF-β), while T_{H2} cells produce interleukin-4 (IL-4), interleukin-5 (IL-5) and interleukin-13 (IL-13), among numerous other cytokines. Interleukin-2 is associated with T_{H1} cells, and its production by helper T cells is necessary for the proliferation of cytotoxic CD8+ T cells.

Given the relative specificity of the cytokines released by either response on particular sections of the immune system, it has been suggested, that both T_{H} groups play separate roles during an immune response. That is, T_{H1} cells are necessary in maximizing the killing efficacy of the macrophages and in the proliferation of cytotoxic CD8+ T cells, therefore their primary role during an immune response is to activate and proliferate these cells.

The above-mentioned finding in a context of the MVA according to the invention is thus of great advantage.

According to a further aspect of the invention the heterologous nucleic acid of the MVA according to the invention is incorporated into a non-essential region of the genome of the MVA.

A modified vaccinia virus Ankara (MVA) is a chicken cell adapted strain of vaccinia virus. Because of its avirulence found upon inoculation of animals and its striking deficiency to produce substantial amounts of new viral progeny in most cells of mammalian origin, MVA can be used under laboratory conditions of biosafety level 1. MVA serves as an efficient vector virus for expression of recombinant genes (Sutter and Moss, 1992) and as candidate recombinant vaccine (Moss et al., 1996) with high safety profiles since MVA has been tested for pre-immunization in over 100000 humans being vaccinated against smallpox without causing notable side-effects. Several MVA vector vaccines have already entered clinical evaluation (McConkey et al., 2003, Cosma et al., 2003). Most recently, MVA is reassessed as candidate second generation vaccine against smallpox. According to the invention, the heterologous nucleic acid according to the invention is incorporated into a non-essential region of the genome of the MVA. According to the present invention, any MVA strain may be used.

WO 03/097844 A1 discloses a number of MVA strains on p. 4. One strain that may be used according to the present invention is the MVA-BN strain or a derivative thereof (WO 02/42480). Non-essential regions according to the present invention may be selected from (i) natural occurring deletion sides of the MVA genome with respect to the genome of the vaccinia virus strain Copenhagen or (ii) intergenic regions of the MVA genome. The term "intergenic region" refers preferably to those parts of the viral genome located between two adjacent genes that comprise neither coding nor regulatory sequences. However, the insertion sides for the incorporation of the heterologous nucleic acid according to the invention (non-essential region) are not restricted to these preferred insertion sides since it is within the scope of the present invention that the integration may be anywhere in the viral genome as long as it is possible to obtain recombinants that can amplified and propagated in at least one cell culture system, such as Chicken Embryo Fibroblasts (CEF cells). Thus, a non-essential region may also be a non-essential gene or genes, the functions of which may be supplemented by the cell system used for propagation of MVA.

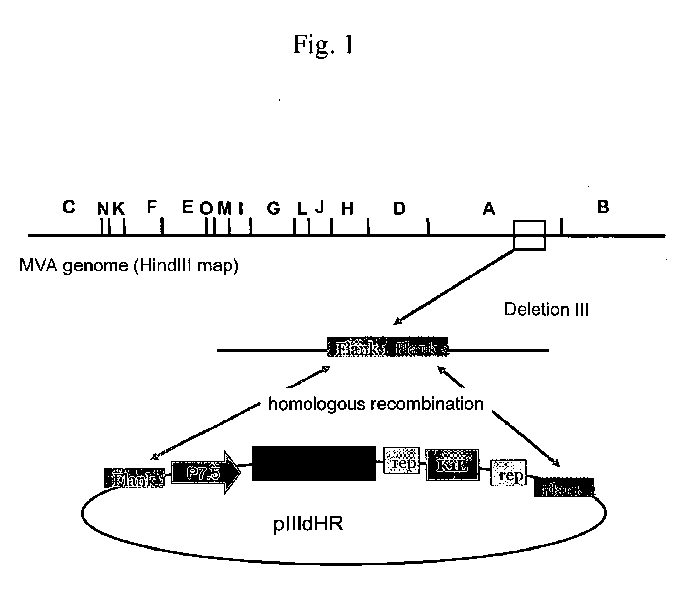

In a particularly preferred embodiment the heterologous nucleic acid according to the invention is incorporated into the MVA genome at the side of deletion (III). Integration of the heterologous nucleic acid according to the invention is performed preferentially by homologous recombination of the flanking regions of a so-called transfer vector, which initially comprises the heterologous nucleic acid according to the invention prior to its integration into the MVA genome. Upon homologous integration selection of recombinant viruses is performed. One such method for selecting the recombinant viruses is expression of the host range gene K1L (see Fig. 1).

According to one embodiment of the present invention the heterologous nucleic acid is under the control of a vaccinia virus-specific promoter, an orthopox virus-specific promoter or a pox virus-specific promoter. A number of promoters may be used for the present invention. For the expression of the heterologous nucleic acid according to the invention several promoters, such the 30K and 40K promoters (US 5,747,324, A Strong Synthetic Early-Late Promoter (Sutter, et al., Vaccine (1994), 12, 1032-1040)), the P7.5 promoter (Endo et al., J. Gen. Virol. (1991) 72, 699-703) and a promoter derived from the cow pox virus type A (inclusion ATI gene) (Lee et al., J. Gen. Virol. 1998), 79, 613). All of these promoters may be used according to the invention. In one embodiment it is desired that the antigen against which an immune response is to be induced is expressed in high amounts. WO 03/097844 A1 discloses such a promoter.

The heterologous nucleic acid according to the invention is selected from the group of nucleic acids encoding an allergen, a mutated allergen, or a fragment of an allergen selected from the group of weed pollens, grass pollens, tree pollens, mites, animals, fungi, insect, rubber, worms, human autoallergens and foods.

Rapid advances have been made in the past two years on allergen characterization in sequence determination by chemical and molecular biological approaches. This is indicated by the list of allergens with known partial or complete amino acid sequence. A useful source for allergen sequences is www.allergen.org. According to the invention, a heterologous nucleic acid of the invention is a nucleic acid encoding an allergen which may lead to a type I hypersensitivity reaction. Allergens are designated according to the accepted taxonomic name of their source as follows: The first three letters of the genus, space, the first letter of the species, space, an Arabic number. The numbers are assigned to the allergens in the order of their identification, and the same number is generally used to designate homologous allergens of related species. As to examples, Lol p 1 refers to the first pollen allergen identified from *Lolium perenne,* rye grass, and Cyn d 1 refers to homologous pollen allergen from *Cynodon dactylon,* Bermuda grass. In some instances, the above system of the first three letters of a genus and the first letter of the species has to be modified to include an additional letter for the designation of the exact genus or species.

An allergen from a single species may consist of several closely similar molecules. These similar molecules are designated as iso-allergens when they share the following biochemical properties: a) similar molecular size; b) identical biological function, if known e.g. enzymatic action; and c) > 67 % identity of amino acid sequences.

It is recognized that the recommended > 67 % sequence identity for two allergens to be assigned to the same group is only a guide. There are likely to be borderline cases. As an example, the rag weed allergens Amb a 1 and 2 share 65 % amino acid sequence identity (Griffith, I. J., J. Pollock, D. G. Klapper, B. L. Rogers, and A. K. Nault. (1991) Sequence Polymorphism of Amb a 1 and Amb a 2, the major allergens in ambrosia artemisii folia (short rag weed) Int. Arch. Allergy Apple. Immunol. 96: 296-304).

cDNA cloning of allergens often show nucleic type mutations which are either silent or which can lead to single or multiple amino acid substitutions. In the revised system, members of allergen group which have > 67 amino acid sequence identity are designated as iso-allergens. Each iso-allergen may have multiple forms of closely similar sequences, which are designated as variants. Table 1 shows some of the allergens according to the present invention.

In a preferred embodiment the heterologous nucleic acid of the present invention encodes an allergen which may not be treated with the specific immunotherapy (SIT). In a particularly preferred embodiment of the present invention the heterologous nucleic acid according to the invention encodes a food allergen such as the food allergen designated in table 1, in particular Gad c 1, Gal d 1, Gal d 2, Gal d 3, Gal d 4, Pen a 1, Pen i 1, Bra j 1, Hor v 1 and Sin a 1. In a particularly preferred embodiment of the present invention the heterologous nucleic acid according to the present invention encodes Gal d 2.

In one aspect of the present invention the nucleic acid according to the present invention which is under the control of a vaccinia virus-specific promoter and incorporated into the MVA genome into a non-essential region, may in fact be the code for more than one allergen.

In one such embodiment such a nucleic acid encodes the most common food allergen, in another embodiment it encodes the most common weed pollens, grass pollens, tree pollens, mites, animals, fungi, insects and others. As known for mixtures used in the specific immunotherapy (SIT), it is thus also an object of this invention to combine allergens into one heterologous nucleic acid within the MVA according to the present invention. One skilled in the art will appreciate that various combinations in particular such combinations are useful, where numerous allergies are found in a majority of the population.

In a preferred embodiment the allergen according to the invention is selected from the group of allergens of table 1.

In a preferred embodiment the allergen according to the invention is selected from the group of hen's-egg ovalbumin (Gal d 2), ovomucoid (Gal d 1), milk, caseins (Bos d 8), β-lactoglubulin (Bos d 5), fish, parvalbumin (Gad c 1) from codfish and homologues from other species, shrimp, tropomyosin (Pen a 1) and homologues from other species including other *Crustacea,* arginine kinase (Pen m 2) and homologous from other species including other *Crustacea,* hazelnut, (Cor a 1.04, Cor a 8) and oleosins and 2S albumins from hazelnut and other kinds of tree nuts (e. g. brazil nut, cashew, almond, peanut in particular, Ara h 1, Ara h 2, Ara h 3) soybean in particular, Gly m 4, glycinin, β-conglycinin, Gly m bd 30 k, fruits in particular lipid transfer proteins and Bet v 1 homologous and rubber latex in particular Hev b 1 to Hev b 11.

In a further preferred embodiment the allergen is selected from the group of grass group I and group V allergens, tree pollen I in particular the Bet v I family, weed pollen in particular Amb a 1, 2, Art v 1, 2 and 3 and homolgues from other species, mites in particular Der p 1, Der f 1, Der p 2, Der f 2, Der p 10, Der f 10, animals in particular Fel d 1 and moulds in particular Alt a 1 to Alt a 13 and homologues from other species.

In one embodiment the nucleic acid according to the invention encodes two or more allergens, fragments or mutants from the group of allergens according to the invention. This means, that the vector used for transfer into MVA as well as later, the MVA according to the invention actually comprises two or more allergens, fragments or mutants. That means that if the nucleic acid according to the invention is transcribed and expressed as a protein a multiple allergens, fragments or mutants may be produced.

In a preferred embodiment the nucleic acid according to the invention encodes two or more homologous allergens, fragments or mutants from different organisms or species.

In a particularly preferred embodiment the nucleic acid according to the invention encodes two or more homologous allergens, fragments or mutants all of which are Bet v 1 homologous comprising homologues such as those selected from the group of Bet v 1 from different pollen such as but not limited to hazel and alder, and Bet v 1 from foods such as but not limited to apple, hazelnut, carrot, celeriac and soybean.

In one embodiment according to the invention the nucleic acid according to the invention encodes two or more homologous allergens, fragments or mutants selected from the group of lipid transfer proteins such as but not limited to pollen, plant derived foods, mugwort pollen, ragweed pollen, plane tree pollen, Parietaria pollen, peach, hazelnut, peanut and wheat.

In a particularly preferred embodiment the allergen is ovalbumin.

The invention also relates to a recombinant modified vaccinia virus Ankara (MVA) comprising a heterologous nucleic acid, wherein the heterologous nucleic acid is incorporated into a non-essential region of the genome of the MVA, the heterologous nucleic acid is under the control of a vaccinia virus-specific promoter, orthopox virus-specific promoter, or a poxvirus-specific promoter and the heterologous nucleic acid is selected from the group of nucleic acids encoding an allergen selected from the group of weed pollens, grass pollens, tree pollens, mites, animals, funghi, insects, rubber, worms, human autoallergens and foods.

As already outlined above, in a preferred embodiment the recombinant modified vaccinia virus Ankara according to the invention comprises a nucleic acid encoding an allergen, fragment or mutant thereof which is selected from the group of allergens of table 1.

In a particularly preferred embodiment the allergen is ovalbumin.

The invention also relates to a nucleic acid encoding a modified vaccinia virus Ankara (MVA).

In a further embodiment the invention relates to a method of introducing a MVA according to the invention into a target cell comprising infection of the target cell with an MVA according to the invention. Viruses are propagated and titered following standard methodology. To generate vaccine preparations, viruses may routinely be purified by ultracentrifugation through sucrose and reconstituted in, e.g. 1 mM Tris pH 9.0. CEF and rabbit kidney RK-13 (ATCC CCL-37) cells may be grown in minimal essential media (MEM) supplemented with 10 % fetal bovine serum (FBS) and maintained at 37°C and 5 % CO₂. CEF cells may be grown in 6 well tissue culture plates and infected with the multiplicity of 0.01 to 20 infectious units MVA.

The determined low or high multiplicity growth profiles, confluent CEF monolayers (grown on 6 well plates) may be infected with 0.05 infectious units (IU) or 10 IU MVA or MVA according to the invention per cell, respectively. After virus absorption for 60 min at 37°C the inoculum may be removed. Cells may be washed twice with RPMI 1640 and incubated with fresh RPMI 1640 medium containing 10 % FCS at 37°C and 5 % CO₂. At multiple time points post infection (p.i.) infected cells may be harvested and virus may be released by freeze-drying and a brief sonication. Serial dilutions of the resulting virus suspension may be plated on confluent CEF monolayers grown in 6 well plates as replicates of two. At 48 hrs p.i. monolayers may be briefly fixed in acetone:methanol (1:1) and cells may be incubated for 60 min with polyclonal rabbit anti-vaccinia antibody (IgG fraction, Biogenesis Ltd., Pool, England), followed by an incubation for 45 min horseradish-peroxidase-conjugated polyclonal goat anti-rabbit antibody (Dianova, Hamburg). After washing with PBS, antibody-labeled cells may be developed using an O-Di-anisidine (Sigma, Taufkirchen, Germany) substrate solution, foci of stained cells may be counted, and virus titers may be calculated as IU/ml.

According to one embodiment of the present invention, the invention relates to a method for immunization of a living animal body including a human, said method comprising administering to said living animal body including a human in need of a therapeutically effective amount of the MVA according to the invention. Said method according to the invention may comprise a composition which may also contain (in addition to the ingredient and the carrier) diluents, common fillers, salts, buffers, stabilizers, solubilizers and other materials well known in the art. It is necessary that these are pharmaceutically acceptable. The term "pharmaceutically acceptable" means a non-toxic material that does not interfere with the effectiveness of the biological activity of the MVA according to the invention. The characteristics of the carrier will depend on the route of administration. The pharmaceutical composition may further contain other agents which either enhance the activity or use in treatment. Such additional factors and/or agents may be included in the pharmaceutical composition to be applied for the method for immunization according to the invention to produce a synergistic effect or to minimize side-effects. Techniques for formulation and administration of the MVA according to the invention may be found in "Remington's Pharmaceutical Sciences", (Muck Publishing Company, Easton, PA, latest edition). The method for immunization according to the present invention will make use of a therapeutically effective amount of the MVA. A therapeutically effective dose further refers to that amount of the compound/ingredient sufficient to result in amelioration of symptoms, e.g. treatment, healing, prevention or amelioration of such conditions. To prepare vaccines, the MVA vaccinia virus generated according to the invention are converted into a physiologically acceptable form. This can be done based on the many years of experience in the preparation of vaccines used for vaccination against smallpox (Kaplan, B. R. Med. Bul. 25, 131-135 [1996]). Typically, about 10⁶ to 10⁷ particles of the recombinant MVA are freeze-dried in 100 ml of phosphate-buffered saline (PBS) in the presence of 2 % peptone and 1 % human albumin in an ampoule, preferably a glass ampoule The lyophilisate can contain extenders, such as manitol, dextran, sugar, glycine, lactose or polydinylpyrolidole or other aids (such as anti-oxidants, stabilizers, etc.) suitable for parenteral administration. The glass ampoule is then sealed and can be stored, preferably at temperatures below -20°C for several months. For vaccination the lyophilisate can be dissolved in 0.1 to 0.2 ml of aqueous solution, preferably physiological saline, and administered parentally, for example by intradermal inoculation. The vaccine according to the invention is preferably injected intracutaneously. Slight swelling and redness, sometimes also itching, may be found at the injection side. The mode of administration, the dose and the number of administrations can be optimized by those skilled in the art in a known manner. It is expedient where appropriate to administer the vaccine several times over a lengthy period in order to obtain a high-level immune response against the foreign antigen. In a preferred embodiment the immunizing may be both prophylactic and/or therapeutic. In a further preferred embodiment an allergen may be administered after administration of the MVA according to the invention. In a particularly preferred embodiment the allergen to be administered after the MVA is the same allergen as encoded by the heterologous nucleic acid of the MVA.

### Detailed description of the figures

### Fig. 1: Construction of a recombinant MVA according to the invention

After introduction of the OVA-gene into the MVA transfer vector pIIIdHR under the control of the vaccinia virus-specific promoter P7.5 the OVA expression cassette was inserted into deletion III of the MVA genome by means of homologous recombination of the flanking regions (flank 1 and flank 2). Selection of recombinant viruses was performed by expression of the vaccinia virus host range gene K1L.

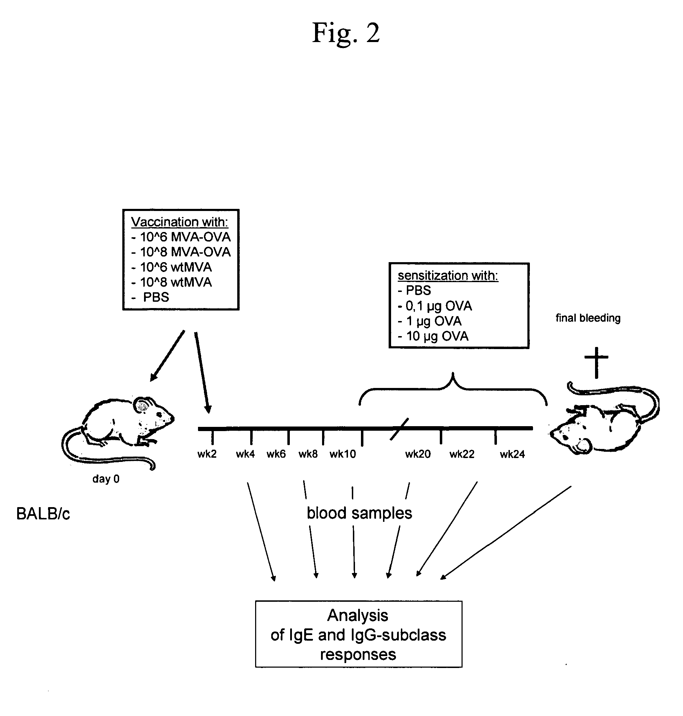

### Fig. 2: Immunization scheme

Female BALB/c mice were vaccinated twice with 10⁶, 10⁸ MVA-OVA or with saline. Sensitizing occurred by intraperitoneal injection of OVA (0.1 µg, 1 µg or 10 µg) or PBS with Al(OH)₃ as adjuvant. The sera taken upon sensitization were tested by means of ELISA and RBL-testing of IgG subclasses and IgE response.

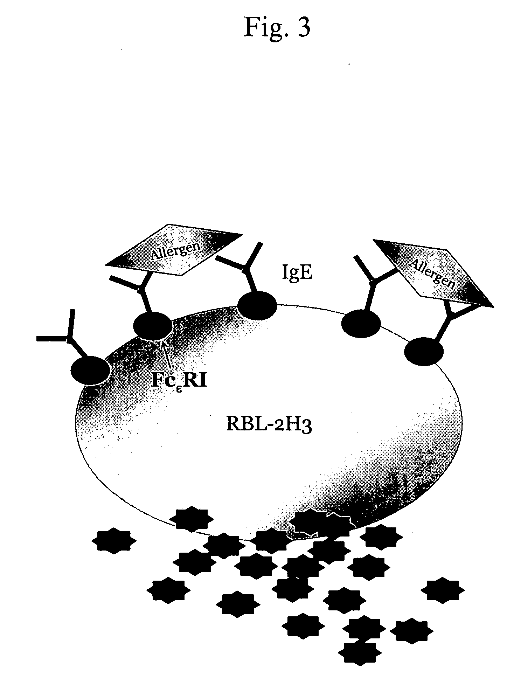

### Fig. 3: Mediator release with rat basophilic leukemia cells (RBL)

Upon incubation of mouse sera, the RBL cells bind IgE by means of the FcεRezeptor on the cell surface. If the respective allergen is added, cross-linking of the antibodies occurs which leads to a degranulation of the cells. The mediator release is quantified by photometric measurement of the β-hexosamidase activity.

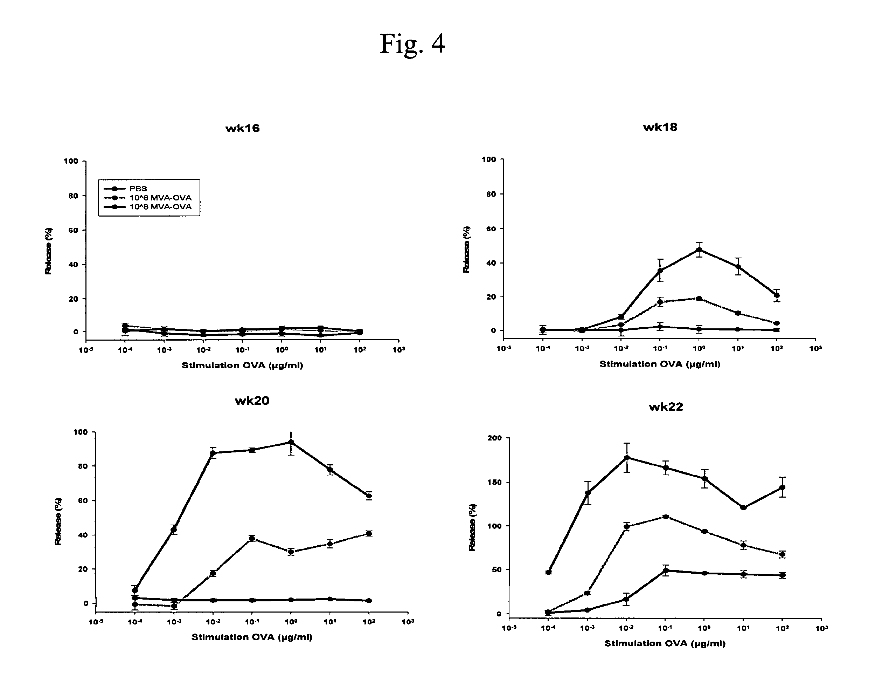

### Fig. 4: Analysis of the IgE response in RBL-assay

Fig. 4 shows an analysis of RBL-assays with sera (pools from mice which were vaccinated with saline (circles), 10⁶ MVA-OVA triangles or 10⁸ MVA-OVA hand which were sensitized with 10 µg OVA. Four time points were tested. The x axis is the concentration of the stimulating antigen (OVA) in the assay.

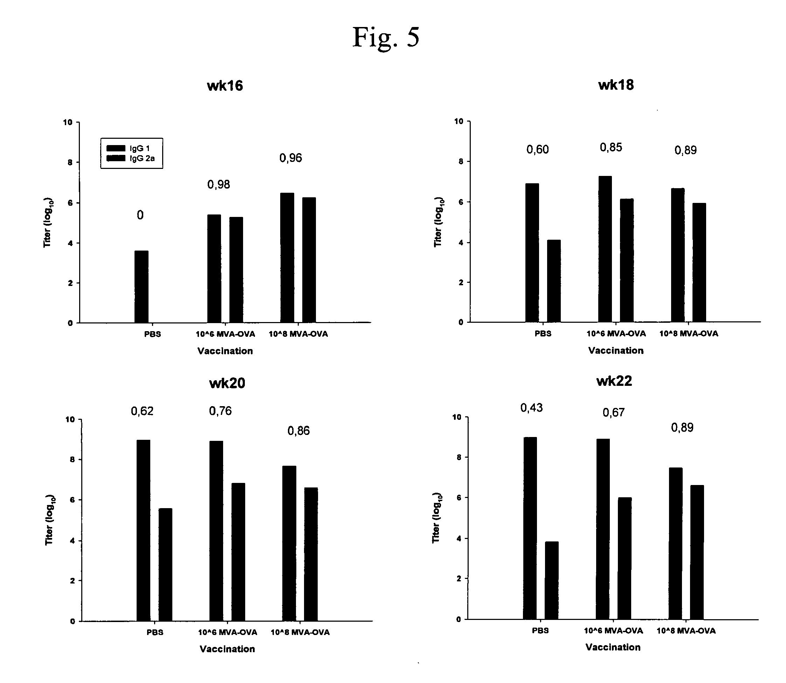

### Fig. 5: Titers of antibodies in pool sera of MVA-OVA or PBS

Vaccinated mice sensitized with 10 µg OVA or analyzed by means of IgG subclass ELISA. The respective ratios of IgG 2A/IgG 1 are shown in the diagram over the bars.

### Examples

The ovalbumin (OVA gene) was inserted under the control of the vaccinia virus-specific P7.5 promoter into the MVA genome (Fig. 1). The resulting construct is designated herein as MVA-OVA. The recombinant MVA-OVA according to the invention was used to infect mice. In mice, the MVA-OVA expressed the ovalbumin allergen under the control of the vaccinia promoter and induced an immune response against OVA. Female BALB/c mice were immunized in steps of two weeks with either 10⁶ or 10⁸ MVA-OVA. After a pause of about eight weeks sensitizing occurred. The sensitizing was done giving 7 doses of OVA (0,1 µg, 1 µg or 10 µg) with the adjuvant Al(OH)₃ in steps of two weeks (Fig. 2). Using an ELISA test, the titers of the OVA specific IgG subclasses could be determined in the sera of the mice whereas the OVA specific IgE activity was determined in a rat basophilic leukemia cell (RBL) assay (Fig. 3).

The vaccination of the mice with MVA-OVA led to a reduced or deferred production of OVA specific IgE antibodies in comparison to non-vaccinated mice. Additionally, higher doses of MVA-OVA (10⁸ IU) led to a stronger immune modulation which persisted for a longer period of time (Fig. 4). The determination of the OVA-specific IgG1 and IgG2A antibodies in the sera of the mice showed an elevated IgG2A/IgG1 ratio in vaccinated mice. This speaks for a T_{H1} response. This ratio decreases with time which correlates with a sensitization at said time. Also this effect is dose-dependent. The IgG2A/IgG1 ratio of the mice immunized with 10⁸ IU MVA-OVA decreases slower than the mice immunized with 10⁶ IU MVA-OVA. Thus, the vaccination with MVA-OVA leads to an immune response against OVA which protect the mice long before sensitization with the allergen. The IgE response against OVA is much lower in vaccinated mice than in non-vaccinated mice. Most advantageously, the ratio of IgG2A to IgG1 in vaccinated mice speaks for a T_{H1} induced response and thus not for an allergic immune response.

**Table 1**

| Species name | Allergen name | Biochemical id or obsolete name | MW kDa SDS-PAGE | C: cDNA P: peptide sequence | Reference and/or accession number |
|---|---|---|---|---|---|
| A. Weeds **Asterales** *Ambrosia artemisiifolia* | | | | | |
| short ragweed | Amb a 1 | antigen E | 38 | C | 8, 20 |
| | Amb a 2 | antigen K | 38 | C | 8,21 |
| | Amb a 3 | Ra3 | 11 | C | 22 |
| | Amb a 5 | Ra5 | 5 | C | 11, 23 |
| | Amb a 6 | Ra6 | 10 | C | 24, 25 |
| | Amb a 7 | Ra7 | 12 | P | 26 |
| | Amb a 8 | profilin | 14 | C | see list of isoallergens |
| | Amb a 9 | polcalcin | 10 | C | see list of isoallergens |
| | Amb a 10 | polcalcin | 18 | C | AY894659 |
| *Ambrosia trifida* | | | | | |
| giant ragweed | Amb t 5 | Ra5G | 4.4 | C | 9, 10, 27 |
| *Artemisia vulgaris* mugwort | Art v 1 | | 27-29 | C | 28 |
| | Art v 2 | | 35 | P | 28A |
| | Art v 3 | lipid transfer protein | 12 | P | 53 |
| | Art v 4 | profilin | 14 | C | 29 |
| | Art v 5 | polcalcin | 10 | C | AY904434 |
| | Art v 6 | pectate lyase, Amb a 1 homologue | 44 | C | AY904433 |
| *Helianthus annuus* | | | | | |
| sunflower | Hel a 1 | | 34 | | 29A |
| | Hel a 2 | profilin | 15.7 | C | Y15210 |
| | Hel a 3 | lipid transfer | 9 | C | AF529201 |
| *Mercurialis annua* | | protein | | | |
| | Mer a 1 | profilin | 14-15 | C | Y13271 |
| **Caryophyllales** | | | | | |
| *Chenopodium album* lamb's-quarters, | Che a 1 | | 17 | C | AY049012, 29B |
| pigweed, | | | | | |
| white goosefoot | Che a 2 | profilin | 14 | C | AY082337 |
| | Che a 3 | polcalcin | 10 | C | AY082338 |
| *Salsola kali* | | | | | |
| Russian-thistle | Sal k 1 | | 43 | P | 29C |
| | | | | | |

| **Gentianales** | | | | | |
|---|---|---|---|---|---|
| *Catharanthus roseus* | | | | | |
| Rosy periwinkle | Cat r 1 | cyclophilin | 18 | C | X85185 |
| **Lamiales** | | | | | |
| *Plantago lanceolata* | | | | | |
| English plantain | Pla l 1 | | 18 | P | P82242, see list of |
| | | | | | isoallergens |
| **Rosales** | | | | | |
| *Humulus japonicus* | | | | | |
| Japanese hop | Hum j 1 | | | C | AY335187 |
| *Parietaria judaica* | | | | | |
| | Par j 1 | lipid transfer | 15 | C | see list of |
| | | protein 1 | | | isoallergens |
| | Par j 2 | lipid transfer | | C | see list of |
| | | protein 2 | | | isoallergens |
| | Par j 3 | profilin | | C | see list of |
| *Parietaria officinalis* | | | | | isoallergens |
| | Par o 1 | lipid transfer | 15 | | 29D |
| | | protein | | | |

| B. Grasses | | | | | |
|---|---|---|---|---|---|
| **Poales** | | | | | |
| *Cynodon dactylon* | | | | | |
| Bermuda grass | Cyn d 1 | | 32 | C | 30, S83343 |
| | Cyn d 7 | | | C | 31, X91256 |
| | Cyn d 12 | profilin | 14 | C | 31a, Y08390 |
| | Cyn d 15 | | 9 | C | AF517686 |
| | Cyn d 22w | enolase | data | | pending |
| | Cyn d 23 | Cyn d 14 | 9 | C | AF517685 |
| | Cyn d 24 | Pathogenesis- | 21 | P | pending |
| | | related p. | | | |
| *Dactylis glomerata* | | | | | |
| orchard grass | Dac g 1 | AgDg1 | 32 | P | 32 |
| | Dac g 2 | | 11 | C | 33, S45354 |
| | Dac g 3 | | | C | 33A, U25343 |
| | Dac g 5 | | 31 | P | 34 |
| *Festuca pratensis* | | | | | |
| meadow fescue | Fes p 4w | | 60 | - | |
| *Holcus lanatus* | | | | | |
| velvet grass | Hol l1 | | | C | Z27084 |
| *Lolium perenne* | | | | | |
| rye grass | Lol p1 | group I | 27 | C | 35, 36 |
| | Lol p2 | group II | 11 | P | 37, 37A, X73363 |
| | Lol p3 | group III | 11 | P | 38 |
| | Lol p5 | Lol p IX, Lol p Ib | 31/35 | C | 34, 39 |
| | Lol p11 | hom: trypsin | 16 | | 39A |
| | | inhibitor | | | |
| *Phalaris aquatica* | | | | | |
| canary grass | Pha a 1 | | | C | 40, S80654 |
| *Phleum pratense* | | | | | |
| timothy | Phl p1 | | 27 | C | X78813 |
| | Phl p2 | | | C | X75925, 41 |
| | Phl p4 | | | P | 41A, see list of |
| | Phl p5 | Ag25 | 32 | C | isoallergens 42, see list of |
| | Phl p6 | | | C | isoallergens Z27082, 43 |
| | Phl p11 | trypsin inhibitor | 20 | C | AF521563,43A |
| | | hom. | | | |
| | Phl p12 | profilin | | C | X77583,44 |
| | Phl p13 | polygalacturonase | 55-60 | C | AJ238848 |
| *Poa pratensis* | | | | | |
| Kentucky blue grass | Poa p 1 | group I | 33 | P | 46 |
| | Poa p 5 | | 31/34 | C | 34,47 |
| *Sorghum halepense* | | | | | |
| Johnson grass | Sor h 1 | | | C | 48 |

| C. Trees | | | | | |
|---|---|---|---|---|---|
| **Arecales** | | | | | |
| *Phoenix dactylifera* | | | | | |
| date palm | Pho d 2 | profilin | 14.3 | C | Asturias p.c. |
| | | | | | |
| **Fagales** | | | | | |
| *Alnus glutinosa* | | | | | |
| alder | Aln g 1 | | 17 | C | S50892 |
| *Betula verrucosa* | | | | | |
| birch | Bet v 1 | | 17 | C | see list of |
| | | | | | isoallergens |
| | Bet v 2 | profilin | 15 | C | M65179 |
| | Bet v 3 | | | C | X79267 |
| | Bet v 4 | | 8 | C | X87153, S54819 |
| | Bet v 6 | h: isoflavone | 33.5 | C | see list of |
| | | reductase | | | isoallergens |
| | Bet v 7 | cyclophilin | 18 | P | P81531 |
| *Carpinus betulus* | | | | | |
| hornbeam | Car b 1 | | 17 | C | see list of |
| | | | | | isoallergens |
| *Castanea sativa* | | | | | |
| chestnut | Cas s 1 | | 22 | P | 52 |
| | Cas s 5 | chitinase | | | |
| | Cas s 8 | lipid transfer | 9.7 | P | 53 |
| | | protein | | | |
| *Corylus avellana* | | | | | |
| hazel | Cor a 1 | | 17 | C | see list of |
| | | | | | isoallergens |
| | Cor a 2 | profilin | 14 | C | |
| | Cor a 8 | lipid transfer | 9 | C | |
| | | protein | | | |
| | Cor a 9 | 11S globulin-like | 40/? | C | Beyer p.c. |
| | | protein | | | |
| | Cor a 10 | luminal binding | 70 | C | AJ295617 |
| | | prot. | | | |
| | Cor a 11 | 7S vicilin-like prot. | 48 | C | AF441864 |
| *Quercus alba* | | | | | |
| White oak | Que a 1 | | 17 | P | 54 |
| | | | | | |
| **Lamiales** | | | | | |
| Oleaceae | | | | | |
| *Fraxinus excelsior* | | | | | |
| ash | Fra e 1 | | 20 | P | 58A, AF526295 |
| *Ligustrum vulgare* | | | | | |
| privet | Lig v 1 | | 20 | P | 58A |
| *Olea europea* | | | | | |
| olive | Ole e 1 | | 16 | C | 59,60 |
| | Ole e 2 | profilin | 15-18 | C | 60A |
| | Ole e 3 | | 9.2 | | 60B |
| | Ole e 4 | | 32 | P | P80741 |
| | Ole e 5 | superoxide | 16 | P | P80740 |
| | | dismutase | | | |
| | Ole e 6 | | 10 | C | 60C, U86342 |
| | Ole e 7 | | ? | P | 60D, P81430 |
| | Ole e 8 | Ca²⁺-binding | 21 | C | 60E, AF078679 |
| | | protein | | | |
| | Ole e 9 | beta-1,3-glucanase | 46 | C | AF249675 |
| | Ole e 10 | glycosyl hydrolase | 11 | C | 60F, AY082335 |
| | | hom. | | | |
| *Syringa vulgaris* | | | | | |
| lilac | Syr v 1 | | 20 | P | 58A |
| | | | | | |

| **Pinales** | | | | | |
|---|---|---|---|---|---|
| *Cryptomeria japonica* | | | | | |
| sugi | Cry j 1 | | 41-45 | C | 55,56 |
| | Cry j 2 | | | C | 57, D29772 |
| *Cupressus arizonica* | | | | | |
| cypress | Cup a 1 | | 43 | C | A1243570 |
| *Cupressus sempervirens* | | | | | |
| common cypress | Cup s 1 | | 43 | C | see list of |
| | | | | | isoallergens |
| | Cup s 3w | | 34 | C | ref pending |
| *Juniperus ashei* | | | | | |
| mountain cedar | Jun a 1 | | 43 | P | P81294 |
| | Jun a 2 | | | C | 57A, AJ404653 |
| | Jun a 3 | | 30 | P | 57B, P81295 |
| *Juniperus oxycedrus* | | | | | |
| prickly juniper | Jun o 4 | hom: calmodulin | 29 | C | 57C, AF031471 |
| *Juniperus sabinoides* | | | | | |
| mountain cedar | Jun s 1 | | 50 | P | 58 |
| *Juniperus virginiana* | | | | | |
| eastern red cedar | Jun v 1 | | 43 | P | P81825, 58B |
| | | | | | |
| **Platanaceae** | | | | | |
| *Platanus acerifolia* | | | | | |
| London plane tree | Pla a 1 | | 18 | P | P82817 |
| | Pla a 2 | | 43 | P | P82967 |
| | Pla a 3 | lipid transfer | 10 | P | Iris p.c. |
| | | protein | | | |

| D. Mites | | | | | |
|---|---|---|---|---|---|
| *Acarus siro* | | arthropod | | | |
| mite | Aca s 13 | fatty acid binding | 14* | C | AJ006774 |
| | | prot. | | | |
| *Blomia tropicalis* | | | | | |
| mite | Blo t 1 | cysteine protease | 39 | C | AF277840 |
| | Blo t 3 | trypsin | 24* | C | Cheong p.c. |
| | Blo t 4 | alpha amylase | 56 | C | Cheong p.c. |
| | Blo t 5 | | | C | U59102 |
| | Blo t 6 | chymotrypsin | 25 | C | Cheong p.c. |
| | Blo t 10 | tropomyosin | 33 | C | 61 |
| | Blo t 11 | paramyosin | 110 | C | AF525465,61A |
| | Blo t 12 | Btlla | | C | U27479 |
| | Blo t 13 | Bt6, fatty acid bind | | C | U58106 |
| | | prot. | | | |
| | Blo t 19 | anti-microbial pep. | 7.2 | C | Cheong p.c. |
| | | hom. | | | |
| *Dermatophagoides farinae* | | | | | |
| American house dust | Der f1 | cysteine protease | 25 | C | 69, see list of |
| mite | | | | | isoallergens |
| | Der f2 | | 14 | C | 70, 70A, see list |
| | | | | | of isoallergens |
| | Der f3 | trypsin | 30 | C | 63 |
| | Der f7 | | 24-31 | C | SW:Q26456, 71 |
| | Der f10 | tropomyosin | | C | 72 |
| | Der f11 | paramyosin | 98 | C | 72A |
| | Der f14 | mag3, apolipophorin | | C | D17686 |
| | Der f15 | 98k chitinase | 98 | C | AF178772 |
| | Der f16 | gelsolin/villin | 53 | C | 71A |
| | Der f17 | Ca binding EF | 53 | C | 71A |
| | | protein | | | |
| | Der f18w | 60k chitinase | 60 | C | Weber p.c. |
| *Dermatophagoides microceras* | | | | | |
| house dust mite | Der m 1 | cysteine protease | 25 | P | 68 |
| *Dermatophagoides pteronyssinus* | | | | | |
| European house dust | Der p 1 | antigen P1, cysteine | 25 | C | 62, see list of |
| mite | | protease | | | isoallergens |
| | Der p 2 | | 14 | C | 62A-C, see list of |
| | | | | | isoallergens |
| | Der p 3 | trypsin | 28/30 | C | 63 |
| | Der p 4 | amylase | 60 | P | 64 |
| | Der p 5 | | 14 | C | 65 |
| | Der p 6 | chymotrypsin | 25 | P | 66 |
| | Der p 7 | | 22/28 | C | 67 |
| | Der p 8 | glutathione | | C | 67A |
| | | transferase | | | |
| | Der p 9 | collagenolytic serine | | P | 67B |
| | | pro. | | | |
| | Der p 10 | tropomyosin | 36 | C | Y14906 |
| | Der p 11 | paramyosin | 103 | C | AY189697, 67C |
| | Der p 14 | apolipophorin like | | C | Epton p.c. |
| | | prot. | | | |
| | Der p 20 | arginine kinase | 40* | C | Thomas p.c. |
| | Der p 21 | | 14 | C | DQ354124 |
| *Euroglyphus maynei* | | | | | |
| mite | Eur m 2 | | | C | see list of |
| | | | | | isoallergens |
| | Eur m 14 | apolipophorin | 177 | C | AF149827 |
| *Glycyphagus domesticus* | | | | | |
| storage mite | Gly d 2 | | | C | 72B, see |
| | | | | | isoallergen list |
| *Lepidoglyphus destructor* | | | | | |
| storage mite | Lep d 2 | Lep d 1 | 15 | C | 73, 74, 74A, see |
| | | | | | isoallergen list |
| | Lep d 5 | | | C | 75, AJ250278 |
| | Lep d 7 | | | C | 75, AJ271058 |
| | Lep d 10 | tropomyosin | | C | 75A, AJ250096 |
| | Lep d 13 | fatty-acid binding | | C | 75, AJ250279 |
| | | protein | | | |
| *Tyrophagus* | | | | | |
| *putrescentiae* | | | | | |
| storage mite | Tyr p 2 | | | C | 75B, Y12690 |
| | Tyr p 13 | fatty-acid binding | 15 | C | AY710432 |
| | | protein | | | |

| E. Animals | | | | | |
|---|---|---|---|---|---|
| *Bos domesticus* | | | | | |
| domestic cattle | Bos d 2 | Ag3, lipocalin | 20 | C | 76, see isoallergen |
| | | | | | list |
| (see also foods) | Bos d 3 | Ca-binding S100 | 11 | C | L39834 |
| | | hom. | | | |
| | Bos d 4 | alpha-lactalbumin | 14.2 | C | M18780 |
| | Bos d 5 | beta-lactoglobulin | 18.3 | C | X14712 |
| | Bos d 6 | serum albumin | 67 | C | M73993 |
| | Bos d 7 | immunoglobulin | 160 | | 77 |
| | Bos d 8 | caseins | 20-30 | | 77 |
| *Canis familiaris* | | | | | |
| *(Canis domesticus)* | Can f1 | | 25 | C | 78, 79 |
| dog | Can f2 | | 27 | C | 78,79 |
| | Can f3 | albumin | | C | S72946 |
| | Can f4 | | 18 | P | A59491 |
| *Equus caballus* | | | | | |
| domestic horse | Equ c 1 | lipocalin | 25 | C | U70823 |
| | Equ c 2 | lipocalin | 18.5 | P | 79A, 79B |
| | Equ c 3 | Ag3 - albumin | 67 | C | 79C, X74045 |
| | Equ c 4 | | 17 | P | 79D |
| | Equ c 5 | AgX | 17 | P | Goubran Botros |
| | | | | | p.c. |
| *Felis domesticus* | | | | | |
| cat (saliva) | Fel d 1 | cat-1 | 38 | C | 15 |
| | Fel d 2 | albumin | | C | 79E, X84842 |
| | Fel d 3 | cystatin | 11 | C | 79F, AF238996 |
| | Fel d 4 | lipocalin | 22 | C | AY497902 |
| | Fel d 5w | immunoglobulin A | 400 | | Adedoyin p.c. |
| | Fel d 6w | immunoglobulin M | 800- | | Adedoyin p.c. |
| | | | 1000 | | |
| | Fel d 7w | immunoglobulin G | 150 | | Adedoyin p.c. |
| *Cavia porcellus* | | | | | |
| guinea pig | Cav p 1 | lipocalin | 20 | P | SW:P83507,80 |
| | | homologue | | | |
| | Cav p 2 | | 17 | P | SW:P83508 |
| *Mus musculus* | | | | | |
| mouse (urine) | Mus m 1 | MUP | 19 | C | 81,81A |
| *Rattus norvegius* | | | | | |
| rat (urine) | Rat n 1 | | 17 | C | 82, 83 |
| | | | | | |

| F. Fungi (moulds) | | | | | |
|---|---|---|---|---|---|
| **1. Ascomycota** | | | | | |
| 1.1 Dothideales | | | | | |
| *Alternaria alternata* | | | | | |
| | Alt a 1 | | 28 | C | U82633 |
| | Alt a 3 | heat shock prot. 70 | | C | U87807, U87808 |
| | Alt a 4 | prot. | 57 | C | X84217 |
| | | disulfideisomerase | | | |
| | Alt a 5 | acid ribosomal | 11 | C | X78222, U87806 |
| | | prot. P2 | | | |
| | Alt a 6 | enolase | 45 | C | U82437 |
| | Alt a 7 | YCP4 protein | 22 | C | X78225 |
| | Alt a 8 | mannitol | 29* | C | AY191815 |
| | | dehydrogenase | | | |
| | Alt a 10 | aldehyde | 53 | C | X78227, P42041 |
| | | dehydrogenase | | | |
| | Alt a 12 | acid ribosomal | 11 | C | X84216 |
| | | prot. P1 | | | |
| | Alt a 13 | glutathione-S- | 26 | C | AY514673 |
| | | transferase | | | |
| *Cladosporium herbarum* | | | | | |
| | Cla h 2 | Ag54 | 23 | | 83B, 83C |
| | Cla h 5 | acid ribosomal | 11 | C | X78223 |
| | | prot. P2 | | | |
| | Cla h 6 | enolase | 46 | C | X78226 |
| | Cla h 7 | YCP4 protein | 22 | C | X78224 |
| | Cla h 8 | mannitol | 28* | C | AY191816 |
| | | dehydrogenase | | | |
| | Cla h 9 | vacuolar serine | 55* | C | AY787775 |
| | | protease | | | |
| | Cla h 10 | aldehyde | 53 | C | X78228 |
| | | dehydrogenase | | | |
| | Cla h 12 | acid ribosomal | 11 | C | X85180 |
| | | prot. P1 | | | |
| | | | | | |

| 1.2 Eurotiales | | | | | |
|---|---|---|---|---|---|
| *Aspergillus flavus* | | | | | |
| | Asp f1 13 | alkaline serine | 34 | | 84 |
| | | protease | | | |
| *Aspergillus fumigatus* | | | | | |
| | Asp f 1 | | 18 | C | M83781, 539330 |
| | Asp f 2 | | 37 | C | U56938 |
| | Asp f 3 | peroxisomal | 19 | C | U20722 |
| | | protein | | | |
| | Asp f 4 | | 30 | C | AJ001732 |
| | Asp f 5 | metalloprotease | 40 | C | Z30424 |
| | Asp f 6 | Mn superoxide | 26.5 | C | U53561 |
| | | dismut. | | | |
| | Asp f 7 | | 12 | C | AJ223315 |
| | Asp f 8 | ribosomal prot. P2 | 11 | C | AJ224333 |
| | Asp f 9 | | 34 | C | AJ223327 |
| | Asp f 10 | aspartic protease | 34 | C | X85092 |
| | Asp f 11 | peptidyl-prolyl | 24 | | 84A |
| | | isomeras | | | |
| | Asp f 12 | heat shock prot. | 90 | C | 85 |
| | | P90 | | | |
| | Asp f 13 | alkaline serine | 34 | | 84B |
| | | protease | | | |
| | Asp f 15 | | 16 | C | AJ002026 |
| | Asp f 16 | | 43 | C | g3643813 |
| | Asp f 17 | | | C | AJ224865 |
| | Asp f 18 | vacuolar serine | 34 | | 84C |
| | | protease | | | |
| | Asp f 22w | enolase | 46 | C | AF284645 |
| | Asp f 23 | L3 ribosomal | 44 | C | 85A, AF464911 |
| | | protein | | | |
| | Asp f 27 | cyclophilin | 18* | C | Crameri p.c. |
| | Asp f 28 | thioredoxin | 12* | C | Crameri p.c. |
| | Asp f 29 | thioredoxin | 12* | C | Crameri p.c. |

| *Aspergillus niger* | | | | | |
|---|---|---|---|---|---|
| | Asp n 14 | beta-xylosidase | 105 | C | AF108944 |
| | Asp n 18 | vacuolar serine | 34 | C | 84B |
| | | protease | | | |
| | Asp n 25 | 3-phytase B | 66-100 | C | 85B, P34754 |
| | Asp n ? | | 85 | C | Z84377 |

| *Aspergillus oryzae* | | | | | |
|---|---|---|---|---|---|
| | Asp o 13 | alkaline serine | 34 | C | X17561 |
| | | protease | | | |
| | Asp o 21 | TAKA-amylase A | 53 | C | D00434, M33218 |

| *Penicillium brevicompactum* | | | | | |
|---|---|---|---|---|---|
| | Pen b 13 | alkaline serine | 33 | | 86A |
| | | protease | | | |
| | Pen b 26 | acidic ribosomal | 11 | C | AY786077 |
| | | prot. P1 | | | |
| *Penicillium* | | | | | |
| *chrysogenum* | | | | | |
| (formerly *P. notatum*) | Pen ch 13 | alkaline serine | 34 | | 87 |
| | | protease | | | |
| | Pen ch 18 | vacuolar serine | 32 | | 87 |
| | | protease | | | |
| | Pen ch 20 | N-acetyl | 68 | | 87A |
| | | glucosaminidas | | | |
| *Penicillium citrinum* | | | | | |
| | Pen c 3 | peroxisomal mem. | 18 | | 86B |
| | | prot. | | | |
| | Pen c 13 | alkaline serine | 33 | | 86A |
| | | protease | | | |
| | Pen c 19 | heat shock prot. | 70 | C | U64207 |
| | | P70 | | | |
| | Pen c 22w | enolase | 46 | C | AF254643 |
| | Pen c 24 | elongation factor 1 | | C | AY363911 |
| | | beta | | | |
| *Penicillium oxalicum* | | | | | |
| | Pen o 18 | vacuolar serine | 34 | | 87B |
| | | protease | | | |

| 1.3 Hypocreales | | | | | |
|---|---|---|---|---|---|
| *Fusarium culmorum* | | | | | |
| | Fus c 1 | ribosomal prot. P2 | 11* | C | AY077706 |
| | Fus c 2 | thioredoxin-like | 13* | C | AY077707 |
| | | prot. | | | |

| 1.4 Onygenales | | | | | |
|---|---|---|---|---|---|
| *Trichophyton rubrum* | | | | | |
| | Tri r 2 | | | C | 88 |
| | Tri r 4 | serine protease | | C | 88 |
| *Trichophyton tonsurans* | | | | | |
| | Tri t 1 | | 30 | P | 88A |
| | Tri t 4 | serine protease | 83 | C | 88 |

| 1.5 Saccharomycetales | | | | | |
|---|---|---|---|---|---|
| *Candida albicans* | | | | | |
| | Cand a 1 | | 40 | C | 89 |
| | Cand a 3 | peroxisomal | 29 | C | AY136739 |
| | | protein | | | |
| *Candida boidinii* | | | | | |
| | Cand b 2 | | 20 | C | J04984, J04985 |

| **2. Basidiomycotina** | | | | | |
|---|---|---|---|---|---|
| 2.1 Hymenomycetes | | | | | |
| *Psilocybe cubensis* | | | | | |
| | Psi c 1 | | | | |
| | Psi c 2 | cyclophilin | 16 | | 89A |
| *Coprinus comatus* | | | | | |
| shaggy cap | Cop c 1 | leucine zipper | 11 | C | AJ132235 |
| | | protein | | | |
| | Cop c 2 | | | | AJ242791 |
| | Cop c 3 | | | | AJ242792 |
| | Cop c 5 | | | | AJ242793 |
| | Cop c 7 | | | | AJ242794 |

| 2.2 Urediniomycetes | | | | | |
|---|---|---|---|---|---|
| *Rhodotorula* | | | | | |
| *mucilaginosa* | | | | | |
| | Rho m 1 | enolase | 47 | C | 89B |
| | Rho m 2 | vacuolar serine | 31 | C | AY547285 |
| | | protease | | | |

| 2.3 Ustilaginomycetes | | | | | |
|---|---|---|---|---|---|
| *Malassezia furfur* | | | | | |
| | Mala f 2 | MF1, peroxisomal | 21 | C | AB011804, 90 |
| | | membrane protein | | | |
| | Mala f 3 | MF2, peroxisomal | 20 | C | AB011805, 90 |
| | | membrane protein | | | |
| | Mala f 4 | mitochondrial | 35 | C | AF084828, 90A |
| | | malate | | | |
| | | dehydrogenase | | | |

| *Malassezia sympodialis* | | | | | |
|---|---|---|---|---|---|
| | Mala s 1 | | | C | X96486, 91 |
| | Mala s 5 | | 18* | C | AJ011955 |
| | Mala s 6 | | 17* | C | AJ011956 |
| | Mala s 7 | | | C | AJ011957,91A |
| | Mala s 8 | | 19* | C | AJ011958,91A |
| | Mala s 9 | | 37* | C | AJ011959, 91A |
| | Mala s 10 | heat shock prot. 70 | 86 | C | AJ428052 |
| | Mala s 11 | Mn superoxide | 23 | C | AJ548421 |
| | | dismut. | | | |
| | Mala s 12 | glucose-methanol- | 67 | C | AJ871960 |
| | | choline (GMC) | | | |
| | | oxidoreductase | | | |
| | Mala s 13 | thioredoxin | 12* | C | Crameri p.c. |
| | | | | | |

| **3. Deuteromycotina** | | | | | |
|---|---|---|---|---|---|
| 3.1 Tuberculariales | | | | | |
| *Epicoccum* | | | | | |
| *purpurascens* | | | | | |
| (formerly *E. nigrum*) | Epi p 1 | serine protease | 30 | P | SW:P83340, 91B |

| G. Insects | | | | | |
|---|---|---|---|---|---|
| *Aedes aegyptii* | | | | | |
| mosquito | Aed a 1 | apyrase | 68 | C | L12389 |
| | Aed a 2 | | 37 | C | M33157 |
| *Apis mellifera* | | | | | |
| honey bee | Api m 1 | phospholipase A2 | 16 | C | 92 |
| | Api m 2 | hyaluronidase | 44 | C | 93 |
| | Api m 4 | melittin | 3 | C | 94 |
| | Api m 6 | | 7-8 | P | Kettner p.c. |
| | Api m 7 | CUB serine | 39 | C | AY127579 |
| | | protease | | | |
| *Bombus pennsylvanicus* | | | | | |
| bumble bee | Bom p 1 | phospholipase | 16 | P | 95 |
| | Bom p 4 | protease | | P | 95 |
| *Blattella germanica* | | | | | |
| German cockroach | Bla g 1 | Bd90k | | C | |
| | Bla g 2 | aspartic protease | 36 | C | 96 |
| | Bla g 4 | calycin | 21 | C | 97 |
| | Bla g 5 | glutathione | 22 | C | 98 |
| | | transferase | | | |
| | Bla g 6 | troponin C | 27 | C | 98, see list of |
| | | | | | isoallergens |
| | Bla g 7 | tropomyosin | 40 | C | AF260897 |
| | Bla g 8 | myosin, light chain | | | |
| *Periplaneta americana* | | | | | |
| American cockroach | Per a 1 | Cr-PII | | C | |
| | Per a 3 | Cr-PI | 72-78 | C | 98A |
| | Per a 6 | troponin C | 17 | C | AY792950 |
| | Per a 7 | tropomyosin | 37 | C | Y14854 |
| *Chironomus kiiensis* | | | | | |
| midge | Chi k 10 | tropomyosin | 32.5* | C | AJ012184 |
| *Chironomus thummi thummi* | | | | | |
| midge | Chi t 1-9 | hemoglobin | 16 | C | 99 |
| | Chi t 1.01 | component III | 16 | C | P02229 |
| | Chi t 1.02 | component IV | 16 | C | P02230 |
| | Chi t 2.0101 | component I | 16 | C | P02221 |
| | Chi t 2.0102 | component IA | 16 | C | P02221 |
| | Chi t 3 | component II-beta | 16 | C | P02222 |
| | Chi t 4 | component IIIA | 16 | C | P02231 |
| | Chi t 5 | component VI | 16 | C | P02224 |
| | Chi t 6.01 | component VIIA | 16 | C | P02226 |
| | Chi t 6.02 | component IX | 16 | C | P02223 |
| | Chi t 7 | component VIIB | 16 | C | P02225 |
| | Chi t 8 | component VIII | 16 | C | P02227 |
| | Chi t 9 | component X | 16 | C | P02228 |
| *Ctenocephalides felis felis* | | | | | |
| cat flea | Cte f1 | | | | |
| | Cte f2 | Mlb | 27 | C | AF231352 |
| | Cte f 3 | | 25 | C | |
| *Thaumetopoea* | | | | | |
| *pityocampa* | | | | | |
| pine processionary moth | Tha p1 | | 15 | P | PIR:A59396,99A |
| *Lepisma saccharina* | | | | | |
| silverfish | Lep s 1 | tropomyosin | 36 | C | AJ309202 |
| *Dolichovespula* | | | | | |
| *maculata* | | | | | |
| white face hornet | Dol m 1 | phospholipase A1 | 35 | C | 100 |
| | Dol m 2 | hyaluronidase | 44 | C | 101 |
| | Dol m 5 | antigen 5 | 23 | C | 102, 103 |
| *Dolichovespula arenaria* | | | | | |
| yellow hornet | Dol a 5 | antigen 5 | 23 | C | 104 |
| *Polistes annularies* | | | | | |
| wasp | Pol a 1 | phospholipase A1 | 35 | P | 105 |
| | Pol a 2 | hyaluronidase | 44 | P | 105 |
| | Pol a 5 | antigen 5 | 23 | C | 104 |
| *Polistes dominulus* | | | | | |
| Mediterranean paper | Pol d 1 | | | | Hoffman p.c. |
| wasp | | | | | |
| | Pol d 4 | serine protease | 32-34 | C | Hoffman p.c. |
| | Pol d 5 | | | | P81656 |
| *Polistes exclamans* | | | | | |
| wasp | Pole 1 | phospholipase A1 | 34 | P | 107 |
| | Pole 5 | antigen 5 | 23 | C | 104 |
| *Polistes fuscatus* | | | | | |
| wasp | Pol f5 | antigen 5 | 23 | C | 106 |
| *Polistes gallicus* | | | | | |
| wasp | Pol g 5 | antigen 5 | 24 | C | P83377 |
| *Polistes metricus* | | | | | |
| wasp | Pol m 5 | antigen 5 | 23 | C | 106 |
| *Vespa crabo* | | | | | |
| European hornet | Vesp c 1 | phospholipase | 34 | P | 107 |
| | Vesp c 5 | antigen 5 | 23 | C | 106 |
| *Vespa mandarina* | | | | | |
| giant asian hornet | Vesp m 1 | | | | Hoffman p.c. |
| | Vesp m 5 | | | | P81657 |
| *Vespula flavopilosa* | | | | | |
| yellowjacket | Ves f 5 | antigen 5 | 23 | C | 106 |
| *Vespula germanica* | | | | | |
| yellowjacket | Ves g 5 | antigen 5 | 23 | C | 106 |
| *Vespula maculifrons* | | | | | |
| yellowjacket | Ves m 1 | phospholipase A1 | 33.5 | C | 108 |
| | Ves m 2 | hyaluronidase | 44 | P | 109 |
| | Ves m 5 | antigen 5 | 23 | C | 104 |
| *Vespula pennsylvanica* | | | | | |
| yellowjacket | Ves p 5 | antigen 5 | 23 | C | 106 |
| *Vespula squamosa* | | | | | |
| yellowjacket | Ves s 5 | antigen 5 | 23 | C | 106 |
| *Vespula vidua* | | | | | |
| wasp | Ves vi 5 | antigen 5 | 23 | C | 106 |
| *Vespula vulgaris* | | | | | |
| yellowjacket | Ves v 1 | phospholipase A1 | 35 | C | 105A |
| | Ves v 2 | hyaluronidase | 44 | P | 105A |
| | Ves v 5 | antigen 5 | 23 | C | 104 |
| *Myrmecia pilosula* | | | | | |
| Australian jumper ant | Myr p 1 | | | C | X70256 |
| | Myr p 2 | | | C | S81785 |
| *Solenopsis geminata* | | | | | |
| tropical fire ant | Sol g 2 | | | | Hoffman p.c. |
| | Sol g 4 | | | | Hoffman p.c. |
| *Solenopsis invicta* | | | | | |
| fire ant | Sol i 2 | | 13 | C | 110, 111 |
| | Sol i 3 | | 24 | C | 110 |
| | Sol i 4 | | 13 | C | 110 |
| *Solenopsis saevissima* | | | | | |
| Brazilian fire ant | Sol s 2 | | | | Hoffman p.c. |
| *Triatoma protracta* | | | | | |
| California kissing bug | Tria p 1 | Procalin | 20 | C | AF179004, 111A. |
| | | | | | |

| H. Foods | | | | | |
|---|---|---|---|---|---|
| *Gadus callarias* | | | | | |
| cod | Gad c 1 | allergen M | 12 | C | 112, 113 |
| *Salmo salar* | | | | | |
| Atlantic salmon | Sal s 1 | parvalbumin | 12 | C | X97824 |
| *Bos domesticus* | | | | | |
| domestic cattle | Bos d 4 | alpha-lactalbumin | 14.2 | C | M18780 |
| (milk) | Bos d 5 | beta-lactoglobulin | 18.3 | C | X14712 |
| see also animals | Bos d 6 | serum albumin | 67 | C | M73993 |
| | Bos d 7 | immunoglobulin | 160 | | 77 |
| | Bos d 8 | caseins | 20-30 | | 77 |

| *Gallus domesticus* | | | | | |
|---|---|---|---|---|---|
| chicken | Gal d 1 | ovomucoid | 28 | C | 114, 115 |
| | Gal d 2 | ovalbumin | 44 | C | 114, 115 |
| | Gal d 3 | Ag22, conalbumin | 78 | C | 114, 115 |
| | Gal d 4 | lysozyme | 14 | C | 114, 115 |
| | Gal d 5 | serum albumin | 69 | C | X60688 |

| *Metapenaeus ensis* | | | | | |
|---|---|---|---|---|---|
| shrimp | Met e 1 | tropomyosin | | C | U08008 |

| *Penaeus aztecus* | | | | | |
|---|---|---|---|---|---|
| shrimp | Pen a 1 | tropomyosin | 36 | P | 116 |

| *Penaeus indicus* | | | | | |
|---|---|---|---|---|---|
| shrimp | Pen i 1 | tropomyosin | 34 | C | 116A |

| *Penaeus monodon* | | | | | |
|---|---|---|---|---|---|
| black tiger shrimp | Pen m 1 | tropomyosin | 38 | C | |
| | Pen m 2 | arginine kinase | 40 | C | AF479772, 117 |

| *Todarodes pacificus* | | | | | |
|---|---|---|---|---|---|
| squid | Tod p 1 | tropomyosin | 38 | P | 117A |
| *Helix aspersa* | | | | | |
| brown garden snail | Hel as 1 | tropomyosin | 36 | C | Y14855, 117B |

| *Haliotis midae* | | | | | |
|---|---|---|---|---|---|
| abalone | Hal m 1 | | 49 | | 117C |
| *Rana esculenta* | | | | | |
| edible frog | Ran e 1 | parvalbumin alpha | 11.9* | C | AJ315959 |
| | Ran e 2 | parvalbumin beta | 11.7* | C | AJ414730 |

| *Brassica juncea* | | | | | |
|---|---|---|---|---|---|
| oriental mustard | Bra j 1 | 2S albumin | 14 | C | 118 |
| *Brassica napus* | | | | | |
| rapeseed | Bra n 1 | 2S albumin | 15 | P | 118A, P80208 |
| *Brassica oleracea* | | | | | |
| cabbage (and others) | Bra o 3 | lipid transfer | 9 | P | Palacin p.c. |
| | | protein | | | |

| *Brassica rapa* | | | | | |
|---|---|---|---|---|---|
| turnip | Bra r 1 | 2S albumin | 10, 14 | C | CAA46782 |
| | Bra r 2 | hom: prohevein | 25 | | P81729 |
| *Hordeum vulgare* | | | | | |
| barley | Hor v 15 | BMAI-1 I | 15 | C | 119 |
| | Hor v 16 | alpha-amylase | | | |
| | Hor v 17 | beta-amylase | | | |
| | Hor v 21 | gamma-3 hordein | 34 | C | 119A, |
| | | | | | SW:P80198 |

| *Secale cereale* | | | | | |
|---|---|---|---|---|---|
| rye | Sec c 20 | secalin | | | see isoall. list |

| *Triticum aestivum* | | | | | |
|---|---|---|---|---|---|
| wheat | Tri a 18 | agglutinin | | | |
| | Tri a 19 | omega-5 gliadin | 65 | P | PIR:A59156 |
| | Tri a 25 | thioredoxin | 13* | C | AJ404845 |
| | Tri a 26 | glutenin | 88 | C | X12928 |

| *Zea mays* | | | | | |
|---|---|---|---|---|---|
| maize, corn | Zea m 14 | lipid transfer prot. | 9 | P | P19656 |
| | Zea m 25 | thioredoxin | 14* | C | AJ890020 |

| *Oryza sativa* | | | | | |
|---|---|---|---|---|---|
| rice | Ory s 1 | | | C | 119B, U31771 |

| *Apium graveolens* | | | | | |
|---|---|---|---|---|---|
| celery | Api g 1 | hom: Bet v 1 | 16* | C | Z48967 |
| | Api g 4 | profilin | | | AF129423 |
| | Api g 5 | | 55/58 | P | P81943 |

| *Daucus carota* | | | | | |
|---|---|---|---|---|---|
| carrot | Dau c 1 | hom: Bet v 1 | 16 | C | 117D, see |
| | | | | | isoallergen list |
| | Dau c 4 | profilin | | C | AF456482 |

| *Corylus avellana* | | | | | |
|---|---|---|---|---|---|
| hazelnut | Cor a 1.04 | hom: Bet v 1 | 17 | C | see list of |
| | | | | | isoallergens |
| | Cor a 2 | profilin | 14 | C | AF327622 |
| | Cor a 8 | lipid transfer | 9 | C | AF329829 |
| | | protein | | | |

| *Fragaria ananassa* | | | | | |
|---|---|---|---|---|---|
| strawberry | Fra a 1 | hom: Bet v 1 | 18 | P | SwissProt: |
| | | | | | Q5ULZ4 |
| | Fra a 3 | lipid transfer | 10 | C | see list of |
| | | protein | | | isoallergens |
| | Fra a 4 | profilin | 13 | C | DR027057 |

| *Malus domestica* | | | | | |
|---|---|---|---|---|---|
| apple | Mal d 1 | hom: Bet v 1 | | C | see list of |
| | | | | | isoallergens |
| | Mal d 2 | hom: thaumatin | | C | AJ243427 |
| | Mal d 3 | lipid transfer | 9 | C | Pastorello p.c. |
| | | protein | | | |
| | Mal d 4 | profilin | 14.4* | C | see list of |
| | | | | | isoallergens |

| *Pyrus communis* | | | | | |
|---|---|---|---|---|---|
| pear | Pyr c 1 | hom: Bet v 1 | 18 | C | AF05730 |
| | Pyr c4 | profilin | 14 | C | AF129424 |
| | Pyr c 5 | hom: isoflavone | 33.5 | C | AF071477 |
| | | reductas | | | |

| *Persea americana* | | | | | |
|---|---|---|---|---|---|
| avocado | Pers a 1 | endochitinase | 32 | C | Z78202 |
| *Prunus armeniaca* | | | | | |
| apricot | Pru ar 1 | hom: Bet v 1 | | C | U93165 |
| | Pru ar 3 | lipid transfer | 9 | P | |
| | | protein | | | |

| *Prunus avium* | | | | | |
|---|---|---|---|---|---|
| sweet cherry | Pru av 1 | hom: Bet v 1 | | C | U66076 |
| | Pru av 2 | hom: thaumatin | | C | U32440 |
| | Pru av 3 | lipid transfer | 10 | C | AF221501 |
| | | protein | | | |
| | Pru av 4 | profilin | 15 | C | AF 129425 |

| *Prunus domestica* | | | | | |
|---|---|---|---|---|---|
| European plum | Pru d 3 | lipid transfer | 9 | P | 119C |
| | | protein | | | |

| *Prunus dulcis* | | | | | |
|---|---|---|---|---|---|
| almond | Pru du 4 | profilin | 14 | C | AY081850, |
| | | | | | AY081852 |

| *Prunus persica* | | | | | |
|---|---|---|---|---|---|
| peach | Pru p 3 | lipid transfer | 10 | P | P81402 |
| | | protein | | | |
| | Pru p 4 | profilin | 14 | C | see isoallergen list |

| *Asparagus officinalis* | | | | | |
|---|---|---|---|---|---|
| asparagus | Aspa o 1 | lipid transfer | 9 | P | 119D |
| | | protein | | | |

| *Crocus sativus* | | | | | |
|---|---|---|---|---|---|
| saffron crocus | Cro s 1 | | 21 | | Varasteh A-R p.c. |
| | Cro s 2 | profilin | 14 | C | AY898658 |

| *Lactuca sativa* | | | | | |
|---|---|---|---|---|---|
| lettuce | Lac s 1 | lipid transfer | 9 | | Vieths p.c. |
| | | protein | | | |

| *Vitis vinifera* | | | | | |
|---|---|---|---|---|---|
| grape | Vit v 1 | lipid transfer | 9 | P | P80274 |
| | | protein | | | |

| *Musa x paradisiaca* | | | | | |
|---|---|---|---|---|---|
| banana | Mus xp 1 | profilin | 15 | C | AF377948 |

| *Ananas comosus* | | | | | |
|---|---|---|---|---|---|
| pineapple | Ana c 1 | profilin | 15 | C | AF377949 |
| | Ana c 2 | bromelain | 22.8* | C | 119E-G, D14059 |
| *Citrus limon* | | | | | |
| lemon | Cit 13 | lipid transfer | 9 | P | Torrejon p.c. |
| | | protein | | | |

| *Citrus sinensis* | | | | | |
|---|---|---|---|---|---|
| sweet orange | Cit s 1 | germin-like protein | 23 | P | Torrejon p.c. |
| | Cit s 2 | profilin | 14 | P | Torrejon p.c. |
| | Cit s 3 | lipid transfer | 9 | P | Torrejon p.c. |
| | | protein | | | |

| *Litchi chinensis* | | | | | |
|---|---|---|---|---|---|
| litchi | Lit c 1 | profilin | 15 | C | AY049013 |

| *Sinapis alba* | | | | | |
|---|---|---|---|---|---|
| yellow mustard | Sin a 1 | 2S albumin | 14 | C | 120 |

| *Glycine max* | | | | | |
|---|---|---|---|---|---|
| soybean | Gly m 1 | HPS | 7 | P | 120A |
| | Gly m 2 | | 8 | P | A57106 |
| | Gly m 3 | profilin | 14 | C | see list of |
| | | | | | isoallergens |
| | Gly m 4 | (SAM22) PR-10 | 17 | C | X60043, 120B |
| | | prot. | | | |

| *Vigna radiata* | | | | | |
|---|---|---|---|---|---|
| mung bean | Vig r 1 | PR-10 protein | 15 | C | AY792956 |

| *Arachis hypogaea* | | | | | |
|---|---|---|---|---|---|
| peanut | Ara h 1 | vicilin | 63.5 | C | L34402 |
| | Ara h 2 | conglutin | 17 | C | L77197 |
| | Ara h 3 | glycinin | 60 | C | AF093541 |
| | Ara h 4 | glycinin | 37 | C | AF086821 |
| | Ara h 5 | profilin | 15 | C | AF059616 |
| | Ara h 6 | hom: conglutin | 15 | C | AF092846 |
| | Ara h 7 | hom: conglutin | 15 | C | AF091737 |
| | Ara h 8 | PR-10 protein | 17 | C | AY328088 |

| *Lens culinaris* | | | | | |
|---|---|---|---|---|---|
| lentil | Len c 1 | vicilin | 47 | C | see list of |
| | | | | | isoallergens |
| | Len c 2 | seed biotinylated | 66 | P | 120C |
| | | prot. | | | |

| *Pisum savitum* | | | | | |
|---|---|---|---|---|---|
| pea | Pis s 1 | vicilin | 44 | C | see list of |
| | | | | | isoallergens |
| | Pis s 2 | convicilin | 63 | C | pending |

| *Actinidia chinensis* | | | | | |
|---|---|---|---|---|---|
| kiwi | Act c 1 | cysteine protease | 30 | P | P00785 |
| | Act c 2 | thaumatin-like | 24 | P | SW:P81370, 121 |
| | | protein | | | |

| *Capsicum annuum* | | | | | |
|---|---|---|---|---|---|
| bell pepper | Cap a 1 w | osmotin-like | 23 | C | AJ297410 |
| | | protein | | | |
| | Cap a 2 | profilin | 14 | C | AJ417552 |

| *Lycopersicon* | | | | | |
|---|---|---|---|---|---|
| *esculentum* | | | | | |
| tomato | Lyc e 1 | profilin | 14 | C | AJ417553 |
| | Lyc e 2 | b- | 50 | C | see isoallergen list |
| | | fructofuranosidase | | | |
| | Lyc e 3 | lipid transfer prot. | 6 | C | U81996 |

| *Solanum tuberosum* | | | | | |
|---|---|---|---|---|---|
| potato | Sola t 1 | patatin | 43 | P | P15476 |
| | Sola t 2 | cathepsin D | 21 | P | P16348 |
| | | inhibitor | | | |
| | Sola t 3 | cysteine protease | 21 | P | P20347 |
| | | inhibitor | | | |
| | Sola t 4 | aspartic protease | 16+4 | P | P30941 |
| | | inhibitor | | | |

| *Bertholletia excelsa* | | | | | |
|---|---|---|---|---|---|
| Brazil nut | Ber e 1 | 2S albumin | 9 | C | P04403, M17146 |
| | Ber e 2 | 11S globulin seed | 29 | C | AY221641 |
| | | storage protein | | | |

| *Juglans nigra* | | | | | |
|---|---|---|---|---|---|
| black walnut | Jug n 1 | 2S albumin | 19* | C | AY102930 |
| | Jug n 2 | vicilin-like prot. | 56* | C | AY102931 |
| *Juglans regia* | | | | | |
| English walnut | Jug r 1 | 2S albumin | | C | U66866 |
| | Jug r 2 | vicilin | 44 | C | AF066055 |
| | Jug r 3 | lipid transfer | 9 | P | Pastorello |
| | | protein | | | |

| *Anacardium occidentale* | | | | | |
|---|---|---|---|---|---|
| Cashew | Ana o 1 | vicilin-like protein | 50 | C | see isoallergen list |
| | Ana o 2 | legumin-like | 55 | C | AF453947 |
| | | protein | | | |
| | Ana o 3 | 2S albumin | 14 | C | AY081853 |

| *Ricinus communis* | | | | | |
|---|---|---|---|---|---|
| Castor bean | Ric c 1 | 2S albumin | | C | P01089 |
| *Sesamum indicum* | | | | | |
| sesame | Ses i 1 | 2S albumin | 9 | C | 121A, AF240005 |
| | Ses i 2 | 2S albumin | 7 | C | AF091841 |
| | Ses i 3 | 7S vicilin-like | 45 | C | AF240006 |
| | | globulin | | | |
| | Ses i 4 | oleosin | 17 | C | AAG23840 |
| | Ses i 5 | oleosin | 15 | C | AAD42942 |
| | Ses i 6 | 11 S globulin | 52 | C | AF091842 |

| *Cucumis melo* | | | | | |
|---|---|---|---|---|---|
| muskmelon | Cuc m 1 | serine protease | 66 | C | D32206 |
| | Cuc m 2 | profilin | 14 | C | AY271295 |
| | Cuc m 3 | pathogenesis-rel p. | 16* | P | P83834 |
| | | PR-1 | | | |

| *Ziziphus mauritiana* | | | | | |
|---|---|---|---|---|---|
| Chinese-date | Ziz m 1 | class III chitinase | 30 | C | AY839230 |
| | | | | | |

| I. Others | | | | | |
|---|---|---|---|---|---|
| *Anisakis simplex* | | | | | |
| nematode | Ani s 1 | | 24 | P | 121B, A59069 |
| | Ani s 2 | paramyosin | 97 | C | AF173004 |
| | Ani s 3 | tropomyosin | 41 | C | 121C, Y19221 |
| | Ani s 4 | | 9 | P | P83885 |

| *Argas reflexus* | | | | | |
|---|---|---|---|---|---|
| pigeon tick | Arg r 1 | | 17 | C | AJ697694 |

| *Ascaris suum* | | | | | |
|---|---|---|---|---|---|
| worm | Asc s 1 | | 10 | P | 122 |

| *Carica papaya* | | | | | |
|---|---|---|---|---|---|
| papaya | Car p 1 | papain | 23* | C | 122A, M15203 |

| *Dendronephthya nipponica* | | | | | |
|---|---|---|---|---|---|
| soft coral | Den n 1 | | 53 | P | 122B |

| *Hevea brasiliensis* | | | | | |
|---|---|---|---|---|---|
| rubber (latex) | Hev b 1 | elongation factor | 58 | P | 123, 124 |
| | Hev b 2 | 1,3-glucanase | 34/36 | C | 125 |
| | Hev b 3 | | 24 | P | 126, 127 |
| | Hev b 4 | component of | 100- | P | 128 |
| | | microhelix complex | 115 | | |
| | Hev b 5 | | 16 | C | U42640 |
| | Hev b 6.01 | hevein precursor | 20 | C | M36986, p02877 |
| | Hev b 6.02 | hevein | 5 | C | M36986, p02877 |
| | Hev b 6.03 | C-terminal | 14 | C | M36986, p02877 |
| | | fragment | | | |
| | Hev b 7.01 | hom: patatin from | 42 | C | U80598 |
| | | B-serum | | | |
| | Hev b 7.02 | hom: patatin from | 44 | C | AJ223038 |
| | | C-serum | | | |
| | Hev b 8 | profilin | 14 | C | see list of |
| | | | | | isoallergens |
| | Hev b 9 | enolase | 51 | C | AJ132580 |
| | Hev b 10 | Mn superoxide | 26 | C | see list of |
| | | dismut. | | | isoallergens |
| | Hev b 11 | class 1 chitinase | | C | see list of |
| | | | | | isoallergens |
| | Hev b 12 | lipid transfer | 9.3 | C | AY057860 |
| | | protein | | | |
| | Hev b 13 | esterase | 42 | P | P83269 |

| *Homo sapiens* | | | | | |
|---|---|---|---|---|---|
| human autoallergens | Hom s 1 | | 73* | C | Y14314 |
| | Hom s 2 | | 10.3* | C | X80909 |
| | Hom s 3 | | 20.1* | C | X89985 |
| | Hom s 4 | | 36* | C | Y17711 |
| | Hom s 5 | | 42.6* | C | P02538 |
| *Triplochiton scleroxylon* | | | | | |
| obeche | Trip s 1 | class 1 chitinase | 38.5 | P | Kespohl p.c. |

## Claims

1. Use of a recombinant modified vaccinia virus Ankara (MVA) comprising a heterologous nucleic acid for the production of a medicament for the prevention and/or treatment of type I hypersensitivity in a living animal including humans.

2. Use according to claim 1, wherein the medicament is a vaccine for immunotherapy and the immune response following application of the said vaccine to said humans is primarily a T_{H1} response in humans.

3. Use according to claims 1 or 2, wherein the heterologous nucleic acid is incorporated into a non-essential site within the genome of MVA.

4. Use according to claim 3, wherein the heterologous nucleic acid is incorporated into the MVA genome at the site of deletion III.

5. Use according to claims 1 to 4, wherein the heterologous nucleic acid is under the control of a vaccinia virus-, or orthopoxvirus-, or poxvirus-specific promoter.

6. Use according to claims 1 to 5, wherein the heterologous nucleic acid is selected from the group of nucleic acids encoding an allergen a mutated allergen, or a fragment of an allergen selected from the group of weed pollens, grass pollens, tree pollens, mites, animals, fungi, insects, rubber, worms, human autoallergens, and foods.

7. Use according to claim 6, wherein the allergen is selected from the group of allergens of table 1.

8. Use according to claim 7, wherein the allergen is selected from the group of hen's egg ovalbumin (Gal d 2), ovomucoid (Gal d 1), milk, caseins (Bos d 8), beta-lactoglubulin (Bos d 5), fish, parvalbumin (Gad c 1) from codfish and homologues from other species, shrimp tropomyosin (Pen a 1) and homologues from other species including other Crustacea and arthropods, arginine kinase (Pen m 2) and homologues from other species including other Crustacea and arthropods, hazelnut (Cor a 1.04, Cor a 8), and oleosins and 2S albumins from hazelnut and other kinds of tree nuts such as brazil nut, cashew, almond, peanut in particular, Ara h 1, Ara h 2, Ara h 3, soybean in particular Gly m 4, glycinin, beta-conglycinin, Gly m bd 30 k, fruits in particular lipid transfer proteins and Bet v 1 homologues and rubber latex in particular Hev b 1 to Hev b 11.

9. Use according to claims 6 or 7, wherein the allergen is selected from the group of grass group I and group V allergens, tree pollen I in particular the Bet v 1 family, weed pollen in particular Amb a 1, Amb a 2, Art v 1, Art v 2 and Art v 3 and homologues from other species, mites in particular Der p 1, Der f 1, Der p 2, Der f 2, Der p 10, Der f 10, animals in particular Fel d 1 and moulds in particular Alt a 1 to Alt a 13 and homologues from other species.

10. Use according to claims 6 to 9, wherein the nucleic acid encodes two or more allergens, fragments, and mutants from the group of allergens according to claims 6 to 9.

11. Use according to claim 10, wherein the nucleic acid encodes two or more allergens, fragments and mutants from one organism or one species.

12. Use according to claim 10, wherein the nucleic acid encodes two or more homologous allergens, fragments and mutants from different organisms or species.

13. Use according to claim 12, wherein the nucleic acid encodes two or more homologous allergens, fragments, or mutants all of which are Bet v 1 homologous comprising homologous such as those selected from the group of Bet v 1 from different pollen, such as but not limited to hazel and alder, and Bet v 1 from foods such as but not limited to apple, hazelnut, carrot, celeriac and soybean.

14. Use according to claim 11 or 12, wherein the nucleic acid encodes two or more homologous allergens, fragments, and mutants selected from the group of lipid transfer proteins such as but not limited to pollen, plant derived foods, mugwort pollen, ragweed pollen, plane tree pollen, Parietaria pollen, peach, hazelnut, peanut and wheat.

15. Use according to claim 7, wherein the allergen is ovalbumin.

16. Recombinant modified vaccinia virus Ankara (MVA) comprising a heterologous nucleic acid, wherein
a. the heterologous nucleic acid is incorporated into a non-essential site within the genome of MVA,
b. the heterologous nucleic acid is under the control of a vaccinia virus-specific promoter, or orthopox virus-specific promoter, or poxvirus-specific promoter and,
c. the heterologous nucleic acid is selected from the group of nucleic acids encoding an allergen selected from the group of weed pollens, gras pollens, tree pollens, mites, animals, fungi, insects, rubber, worms, human autoallergens, and foods.

17. Recombinant modified vaccinia virus Ankara (MVA) according to claim 16, wherein the allergen is selected from the group of allergens of table 1.

18. Recombinant modified vaccinia virus Ankara (MVA) according to claims 16 or 17, wherein the allergen is selected from the group of hen's egg ovalbumin (Gal d 2), ovomucoid (Gal d 1), milk, caseins (Bos d 8), beta-lactoglubulin (Bos d 5), fish, parvalbumin (Gad c 1) from codfish and homologues from other species, shrimp tropomyosin (Pen a 1) and homologues from other species including other Crustacea and arthropods, arginine kinase (Pen m 2) and homologues from other species including other Crustacea and arthropods, hazelnut (Cor a 1.04, Cor a 8), and oleosins and 2S albumins from hazelnut and other kinds of tree nuts such as brazil nut, cashew, almond, peanut in particular, Ara h 1, Ara h 2, Ara h 3 soybean in particular Gly m 4, glycinin, beta-conglycinin, Gly m bd 30 k, fruits in particular lipid transfer proteins and Bet v 1 homologues and rubber latex in particular Hev b 1 to Hev b 11.

19. Recombinant modified vaccinia virus Ankara (MVA) according to claims 16 or 17, wherein the allergen is selected from the group of grass group I and group V allergens, tree pollen I in particular the Bet v 1 family, weed pollen in particular Amb a 1, 2, Art v 1, 2 and 3 and homologues from other species, mites in particular Der p 1, Der f 1, Der p 2, Der f 2, Der p 10, Der f 10, animals in particular Fel d 1 and moulds in particular Alt a 1 to Alt a 13 and homologues from other species.

20. Recombinant modified vaccinia virus Ankara (MVA) according to claims 16 to 19, wherein the nucleic acid encodes two or more allergens, fragments or mutants.

21. Recombinant modified vaccinia virus Ankara (MVA) according to claims 16 to 19, wherein the nucleic acid encodes two or more allergens, fragments or mutants from one organism or species.

22. Recombinant modified vaccinia virus Ankara (MVA) according to claims 16 to 19, wherein the nucleic acid encodes two or more homologous allergens, fragments or mutants from different organisms or species.

23. Recombinant modified vaccinia virus Ankara (MVA) according to claim 22, wherein the nucleic acid encodes two or more homologous allergens, fragments or mutants, all of which are Bet v 1 homologous comprising homologous such as those selected from the group of Bet v 1 from different pollen, such as but not limited to hazel and alder, and Bet v 1 from foods such as but not limited to apple, hazelnut, carrot, celeriac and soybean.

24. Recombinant modified vaccinia virus Ankara (MVA) according to claim 16, wherein the nucleic acid encodes two or more homologous allergens, fragments or mutants, selected from the group of lipid transfer proteins such as but not limited to from pollen, plant derived foods, mugwort pollen, ragweed pollen, plane tree pollen, Parietaria pollen, peach, hazelnut, peanut and wheat.

25. Recombinant modified vaccinia virus Ankara (MVA) according to claim 16, wherein the allergen is ovalbumin.

26. Nucleic acid encoding a modified vaccinia virus Ankara (MVA) according to any of claims 16 to 25.

27. Method of introducing a recombinant MVA according to any of claims 16 to 25 into a target cell comprising infection of the target cell with a recombinant MVA according to any of claims 16 to 25.

28. Method for immunization of a living animal body including a human said method comprising administering to said living animal body including a human in need thereof a therapeutically effective amount of the MVA according to claims 16 to 25.

29. Method according to claim 28, wherein the immunizing may be both prophylactic and/or therapeutic.

30. Method according to claim 14 and 15, wherein sensitization of said living animal body including a human is performed by administering an allergen, fragment or mutant after administration of the MVA.

31. Method according to claim 30, wherein said allergen is the allergen, fragment or mutant encoded by the heterologous nucleic acid of the MVA.
